# EUROPÄISCHE PATENTSCHRIFT

(11) **EP 3 311 141 B1**
(45) Veröffentlichungstag und Bekanntmachung des Hinweises auf die Patenterteilung: **13.09.2023**
(21) Anmeldenummer: 16740932.5
(22) Anmeldetag: 22.06.2016
(51) Int. Cl.: G01N 21/64, G01N 33/42

(54) **VERFAHREN UND VORRICHTUNG ZUR BESTIMMUNG EINER MATERIALEIGENSCHAFT EINES BITUMENMATERIALS**
METHOD AND DEVICE FOR DETERMINING A MATERIAL PROPERTY OF A BITUMEN MATERIAL
PROCÉDÉ ET DISPOSITIF DE DÉTERMINATION D'UNE PROPRIÉTÉ D'UN MATÉRIAU BITUMINEUX

(30) Priorität: 22.06.2015 AT 505222015
(43) Veröffentlichungstag der Anmeldung: 25.04.2018
(73) Patentinhaber: Technische Universität Wien, 1040 Wien (AT)
(72) Erfinder: HOFKO, Bernhard, 1130 Wien (AT); HOSPODKA, Markus, 2340 Mödling (AT); FÜSSL, Josef, 1100 Wien (AT); EBERHARDSTEINER, Lukas, 1130 Wien (AT); GROTHE, Hinrich, 2102 Bisamberg (AT); HANDLE, Florian, 1030 Wien (AT); GROSSEGGER, Daniel, 5760 Saalfelden (AT); BLAB, Ronald, 2103 Langenzersdorf (AT)
(74) Vertreter: SONN Patentanwälte OG
(86) Internationale Anmeldenummer: PCT/AT2016/050219
(87) Internationale Veröffentlichungsnummer: WO 2016/205847

(56) Entgegenhaltungen:
- DE-A1-102007 055 045
- JP-A- 2005 337 885
- US-A- 4 959 549
- US-A- 5 413 108
- US-A- 5 780 850
- Florian Handle ET AL: "CONFOCAL LASER SCANNING MICROSCOPY -OBSERVATION OF THE MICROSTRUCTURE OF BITUMEN AND ASPHALT CONCRETE", 5th Eurasphalt & Eurobitume Congress, 13-15th June 2012, Istanbul, 1. Juni 2012 (2012-06-01), XP055308872, Gefunden im Internet: URL:http://www.h-a-d.hr/pubfile.php?id=480 [gefunden am 2016-10-10]
- J. Kister ET AL: "Effects of Preheating and Oxidation on Two Bituminous Coals Assessed by Synchronous UV Fluorescence and FTIR Spectroscopy", ENERGY & FUELS, vol. 10, no. 4, 1 January 1996 (1996-01-01), pages 948-957, XP055755871, WASHINGTON, DC, US. ISSN: 0887-0624, DOI: 10.1021/ef950159a

## Beschreibung

Die Erfindung betrifft ein Verfahren zur Bestimmung des Alterungszustands bzw. der Alterungsresistenz eines Bitumenmaterials.

Bitumen und bituminöse Stoffe sind wichtige Rohstoffe in Bauwesen, Industrie und Produktion. Insbesondere in der Bauindustrie werden die Anforderungen an Material und Produkt ständig erhöht, wodurch eine verbesserte Qualitätskontrolle und Analyse der eingesetzten Stoffe notwendig wird. Die gestiegenen Anforderungen beziehen sich insbesondere auf die Alterungsbeständigkeit des Materials, die bisher nur grob in zeitaufwendigen Tests bestimmt werden konnte. Bisher mangelte es vor allem an einer grundlegenden, raschen Prüfung der Alterungsresistenz, die eine Qualitätsprüfung in verschiedenen Schritten des Arbeitsprozesses möglich macht. Wünschenswert wäre insbesondere, Materialeigenschaften des Bitumens im Produktions-, Lager-, und Verarbeitungsprozess zu erfassen, ohne dafür besonderen Zeitaufwand zu verlangen.

Dies betrifft beispielsweise das Recycling von Straßenbaubitumen. Darunter wird die Wiederverwendung von bituminös gebundenem Baustoff verstanden, welcher insbesondere im Straßenbau in großen Mengen anfällt. Die meisten Ausbauasphalte können prinzipiell für die Herstellung von neuem Asphaltmischgut wiederverwendet werden. Die Wiederverwendungsquote von Ausbauasphalt ist jedoch nach wie vor gering. Dies liegt insbesondere daran, dass eine verlässliche Qualitätskontrolle des recycelten Materials fehlt.

Derzeit werden die Alterungserscheinungen des Recyclingasphalts dadurch gemindert, dass sehr weiches, frisches Bindemittel zugegeben wird. Dieses Verfahren ist jedoch nachteiligerweise nicht effizient. Demnach ist es unerlässlich, den Alterungsmechanismus von Bitumen auf chemisch-physikalischer Ebene einzubeziehen, um die Wiederverwendbarkeit von Ausbauasphalt zuverlässig prognostizieren zu können.

Im Stand der Technik wurden hierzu verschiedene Ansätze präsentiert.

In Bezug auf Alterungsmechanismen bei der Langzeitalterung wird häufig auf ein weiterentwickeltes Mizellenmodell für Bitumen zurückgegriffen. Das Mizellenmodell besagt, dass Bitumen aus einer durchgehenden, öligen Phase (Maltenphase) besteht, in der Asphaltenmizellen aus agglomerierten Asphaltenmolekülen dispergiert sind (Emulsion). Damit die hochpolaren Asphaltenmizellen in der niedrigpolaren Maltenphase erfolgreich dispergieren können, braucht es einen Mantel um die Mizellen, der einen Polaritätsgradienten herstellt (Emulgator). Dieser Mantel ist aus Aromaten und Harzen aufgebaut. Beim Angriff durch Oxidationsmittel gelangen diese zunächst in die Maltenphase, die aber kaum reaktiv ist. Erst am Mantel der Partikel beginnt die intensive Oxidation, wodurch es an der Grenzfläche zwischen Mantel und Maltenphase zu einem Anstieg der Polarität kommt. Damit verringert sich die Qualität der Dispersion, es entsteht gleichsam eine Sollbruchstelle an der Grenzfläche, die zu einer Erhöhung von Härte und Sprödigkeit des Materials führt und so die Bildung von Sprödbrüchen begünstigt. Damit kann erklärt werden, wie es mit zunehmender Alterung zur Erhöhung der Sprödigkeit kommt. Bei Untersuchungen der Anmelderin hat sich gezeigt, dass weder die Maltenphase, noch die Asphaltene ihr mechanisches Verhalten infolge der Alterung maßgeblich verändern. Die zunehmende Steifigkeit von Bitumen mit fortschreitender Alterung kann durch den Anstieg der Asphaltenkonzentration in Relation zur Konzentration kleinerer, weniger polarer Aromaten erklärt und modellhaft beschrieben werden.

Zur Untersuchung dieser Vorgänge wurde insbesondere die im Stand der Technik an sich bekannte Fluoreszenzspektroskopie und Fluoreszenzmikroskopie eingesetzt (vgl. Handle, Florian, et al. "Understanding the microstructure of bitumen: a CLSM and fluorescence approach to model bitumen ageing behavior." Proceedings to 12th ISAP International Conference on Asphalt Pavements, Raleigh, USA 2014; Handle et al. "The bitumen microstructure: a fluorescent approach", Materials and Structures, Dezember 2014; Bearsley et al. "Direct observation of the asphaltene structure in paving-grade bitumen using confocal laser-scanning microscopy, Journal of Microscopy, Vol. 215, 2004). Aus diesen Untersuchungen konnte ein besseres Verständnis der Alterung von Bitumen gewonnen werden.

Auch Handle, Florian, et al. "Confocal Laser Scanning Microscopy - Observation of the Microstructure of Bitumen and Asphalt Concrete", 5th Eurasphalt & Eurobitume Congress, 13.-15 Juni 2012, Istanbul, 1. Juni 2012 trägt zum besseren Verständnis von Bitumenmaterial bei.

Im Stand der Technik wurden zudem verschiedenste Apparaturen zur Messung von Fluoreszenz vorgeschlagen (vgl. z. B. CA 2 833 299, US 2005/0253088 A1, US 4,330,207, WO 2010/048584, US 7,633,071 B2, US 2014/0135431 A1).

Daneben ist aus der US 4 959 549 ein System zum Messen bestimmter Eigenschaften von Kohle durch Auswertung eines Fluoreszenzsignals bekannt. Bei dem System wird ein Anregungslicht von einem gepulsten Stickstofflaser mit einer Wellenlänge von 337 nm auf eine Kohleanhäufung auf einem Fördermittel gerichtet. Das Anregungslicht passiert hierbei einen Monochromator, sodass eine Anregung mit weitgehend monochromatischer Strahlung erfolgt. Die entstehende Fluoreszenzstrahlung wird über Linse in einem Photomultiplier erfasst. Zudem ist wiederum ein Doppelmonochromator vor dem Photomultiplier vorgesehen, sodass aus der Fluoreszenzstrahlung bestimmte Wellenlängen erfasst werden können. Allerdings ist auch dieses System nicht dazu geeignet, Alterungszustände von Bitumenmaterial zu erfassen.

Die US 5,780,850 A betrifft die Bestimmung des API Grades von Öl bei Ölbohrungen. Dabei wird eine aufgelöste Probe mittels einer Anregungsstrahlung angeregt, die Intensität an zwei unterschiedlichen Wellenlängen gemessen und in weiterer Folge ein Verhältnis aus den gemessenen Intensitäten gebildet. Schließlich wird daraus der API Grad der Probe bestimmt.

Bislang wurde jedoch keine zufriedenstellende Lösung gefunden, wie die aus der Fluoreszenzspektroskopie gewonnenen theoretischen Erkenntnisse über den Alterungsprozess von Bitumen in ein praxistaugliches Verfahren zur Analyse der Eignung von Ausbauasphalt für die Wiederverwendung umgesetzt werden könnte.

In der JP 2005-337885 A wird auf einem abliegenden Gebiet ein andersartiges Verfahren zur Bestimmung des Abnutzungsgrades eines Kabels mit Hilfe von Fluoreszenzspektroskopie beschrieben. Damit soll der rechtzeitige Austausch des Kabels bei einer gewissen Abnutzung ermöglicht werden. Das Kabelmaterial wird mit einer breitbandigen Anregungsstrahlung angeregt, woraufhin die Verschiebung des Fluoreszenzsignals gemessen wird. Dieses Verfahren ist jedoch weder für die Charakterisierung von Bitumen vorgesehen noch wäre es dafür geeignet.

Die Veröffentlichung J. Kister ET AL: "Effects of Preheating and Oxidation on Two Bituminous Coals Assessed by Synchronous UV Fluorescence and FTIR Spectroscopy", ENERGY & FUELS, Bd. 10, Nr. 4, 1. Januar 1996 (1996-01 -01), Seiten 948-957, betrifft die Untersuchung von Steinkohle mittels Fluoreszenzspektroskopie.

US 5,413,108 A betrifft eine Vorrichtung zur Untersuchung von Gewebe.

Demnach besteht die Aufgabe der Erfindung darin, die Nachteile des Standes der Technik zu lindern bzw. zu beseitigen. Die Erfindung setzt sich daher insbesondere zum Ziel, ein Verfahren zu schaffen, mit welchem der Alterungszustand bzw. die Alterungsresistenz eines Bitumenmaterials rasch und unkompliziert bestimmt werden können.

Diese Aufgabe wird durch ein Verfahren mit den Merkmalen von Anspruch 1 gelöst. Bevorzugte Ausführungsformen sind in den abhängigen Ansprüchen angegeben. In dem Verfahrensanspruch 9 ist eine alternative Lösung derselben Aufgabe für Straßenbaubitumen angegeben.

Das erfindungsgemäße Verfahren weist daher zumindest die folgenden Schritte auf:
- Beaufschlagen des Bitumenmaterials mit einer im Wesentlichen monochromatischen ersten Anregungsstrahlung einer ersten Anregungswellenlänge;
- Messen der Intensität einer durch die erste Anregungsstrahlung angeregten ersten Fluoreszenzstrahlung in einem Messwellenlängenbereich;
- Beaufschlagen des Bitumenmaterials mit einer im Wesentlichen monochromatischen zweiten Anregungsstrahlung einer zweiten Anregungswellenlänge;
- Messen der Intensität einer durch die zweite Anregungsstrahlung angeregten zweiten Fluoreszenzstrahlung in dem Messwellenlängenbereich;
- Ermittlung einer ersten Kennzahl für den Alterungszustand bzw. die Alterungsresistenz des Bitumenmaterials aus dem Verhältnis zwischen der Intensität der zweiten Fluoreszenzstrahlung zu der Intensität der ersten Fluoreszenzstrahlung.

Erfindungsgemäß wird das Bitumenmaterial, insbesondere ein Ausbau- bzw. Recyclingasphalt, einer ersten Anregungsstrahlung mit einer ersten Anregungswellenlänge ausgesetzt, mit welcher insbesondere die fluoreszierenden Zentren des Bitumens zu einer ersten Emission von Fluoreszenzstrahlung angeregt werden, deren Intensität in einem vorgegebenen Messwellenlängenbereich erfasst wird. Vorzugsweise erfolgt die Erfassung der ersten Fluoreszenzstrahlung in einem im Wesentlichen monochromatischen Messwellenlängenbereich, d.h. es wird eine einzelne Messwellenlänge verwendet. Alternativ kann ein Messwellenlängenbereich verwendet werden, welcher im Vergleich zum Wellenlängenbereich, bei dem die erste Fluoreszenzstrahlung auftritt, klein ist. Darüber hinaus wird das Bitumen mit einer zweiten Anregungsstrahlung einer von der ersten Anregungswellenlänge verschiedenen zweiten Anregungswellenlänge bestrahlt. Vorzugsweise erfolgt die Anregung des Bitumenmaterials in einem zeitlichen Abstand nacheinander, um die Auflösung zwischen den Messsignalen der ersten und zweiten Fluoreszenzstrahlung bzw. Fluoreszenzemission zu gewährleisten. Die Intensität der zweiten Fluoreszenzstrahlung wird in demselben Messwellenlängenbereich wie die erste Fluoreszenzstrahlung erfasst. Wie zuvor beschrieben, kann der Messwellenlängenbereich einerseits im Wesentlichen monochromatisch sein. Andererseits kann die Intensität der zweiten Fluoreszenzstrahlung in einem im Vergleich zum gesamten Wellenlängenbereich der zweiten Fluoreszenzstrahlung schmalen Bereich von Messwellenlängen erfasst werden. Schließlich wird eine Kennzahl für die Materialeigenschaft, insbesondere den Alterungszustand bzw. die Alterungsresistenz, des Bitumens aus dem Verhältnis zwischen der Intensität der zweiten Fluoreszenzstrahlung zu der Intensität der ersten Fluoreszenzstrahlung bestimmt. Bei umfangreichen Untersuchungen hat sich insbesondere herausgestellt, dass sich die Alterungsprozesse im Bitumenmaterial auf dessen Fluoreszenzspektrum auswirken. Das erfindungsgemäße Verfahren macht sich diese Erkenntnis zunutze, indem die Intensität der Fluoreszenzstrahlung bei zwei verschiedenen, diskreten Anregungswellenlängen gemessen wird, wobei das Verhältnis der Intensitäten der Fluoreszenzsignale als Kennzahl bzw. Indikationswert insbesondere für den Alterungszustand des Bitumenmaterial herangezogen wird. Vorzugsweise ist die zweite Anregungswellenlänge größer als die erste Anregungswellenlänge. Es konnte nachgewiesen werden, dass die Aufnahme eines vollständigen Anregungsspektrums (engl. "excitation scan") nicht erforderlich ist, um die Alterung des Bitumenmaterials festzustellen. Vielmehr hat es sich als ausreichend erwiesen, wenn die Intensitäten der Fluoreszenzstrahlung bei diskreten (d.h. im Wesentlichen monochromatischen) Anregungsstrahlungen erfasst werden. Die Intensität der ersten Fluoreszenzstrahlung wird hierbei als Referenzgröße herangezogen, auf welche die Intensität der zweiten Fluoreszenzstrahlung bezogen wird. Das Verhältnis zwischen den Intensitätswerten der Fluoreszenzstrahlung bei den verschiedenen Anregungswellenlängen kann ein zuverlässiger Indikator dafür sein, ob sich die Alterungsprozesse negativ auf die Materialeigenschaften des untersuchten Bitumenmaterials ausgewirkt haben. Darüber hinaus kann die erste Kennzahl für eine Erstprüfung herangezogen werden, ob sich mangelnde Alterungsresistenz des Bitumenmaterials auf Grund schlechter Rohstoffqualität, Lagerbedingungen, Verarbeitung, etc. in naher Zukunft deutlich negativ auf die Materialeigenschaften auszuwirken droht. Demnach kann die erste Kennzahl bei einer bevorzugten Anwendung als Maß für die Alterungsbeständigkeit des Bitumenmaterials herangezogen werden, bei welchem es sich insbesondere um einen Ausbau- bzw. Recyclingasphalt handelt. Bei experimentellen Untersuchungen konnte insbesondere gezeigt werden, dass die Alterung des Bitumenmaterials eine Absenkung der Intensität der zweiten Fluoreszenzstrahlung im Verhältnis zur ersten Fluoreszenzstrahlung hervorruft. Somit erlaubt die ermittelte Kennzahl eine Abschätzung des Alterungszustands bzw. der Alterungsresistenz des untersuchten Bitumenmaterials. Je höher die erste Kennzahl ist, desto größer ist die Alterungsbeständigkeit des Bitumenmaterials. Auf Basis der ersten Kennzahl kann somit entschieden werden, ob das Bitumenmaterial für eine Erst-, Wieder- oder Weiterverwendung vorgesehen werden soll. Durch die Verwendung einzelner, im Wesentlichen monochromatischer Anregungssignale kann der konstruktive Aufwand für die Umsetzung des Verfahrens gering gehalten werden. Vorteilhafterweise kann auf die Aufnahme eines Anregungsspektrums über ein breites Intervall von Anregungswellenlängen verzichtet werden. Der Alterungszustand des Bitumenmaterials kann erfindungsgemäß mit einer Mindestzahl von Fluoreszenzmessungen analysiert werden. Dadurch kann auch der Zeitaufwand für das Verfahren wesentlich reduziert werden, wodurch insbesondere eine Echtzeit-Anwendung des Verfahrens, beispielsweise auf einem Handgerät, ermöglicht wird. Um die Bitumenprobe mit der im Wesentlichen monochromatischen Anregungsstrahlung zu beaufschlagen, können einerseits Strahlungsquellen verwendet werden, welche im Wesentlichen monochromatische Strahlung, d.h. Strahlung mit einer sehr geringen Bandbreite, aussenden. Dafür eignen sich insbesondere Leuchtdioden oder Laser. Andererseits können Strahlungsquellen wie Gaslampen verwendet werden, welche eine breitbandige Anregungsstrahlung aussenden, aus der mit Hilfe eines Filters bzw. eines Monochromators die gewünschte, im Wesentlichen monochromatische Anregungsstrahlung, erhalten wird.

Das erfindungsgemäße Verfahren eignet sich besonders für die Implementierung auf einem tragbaren Handgerät, bei dem nur diejenigen Wellenlängen angeregt werden, die auch wesentlich für die Beurteilung des Alterungszustands sind. Damit kann erstmals eine Methode geschaffen werden, mit der flexibel im Labor, auf der Baustelle und selbst im eingebauten Zustand die Alterungsbeständigkeit des eingesetzten Bitumens einfach, rasch und günstig bewertet werden kann.

Wenn die erste Kennzahl mit einem ersten Referenzwert für den Alterungszustand bzw. die Alterungsresistenz des Bitumenmaterials verglichen wird, kann auf besonders einfache Weise die Entscheidung getroffen werden, ob das untersuchte Bitumenmaterial einer Erst- oder Wiederverwendung zugeführt werden kann oder ob die Alterungsresistenz des Bitumenmaterials bereits so beeinträchtigt ist, dass eine Erst- oder Wiederverwendung des Bitumenmaterials nicht zielführend wäre, da in naher Zeit starke Schädigungen durch Materialalterung zu erwarten sind. Demnach kann eine mangelnde Qualität des Bitumenmaterials hinsichtlich des Alterungszustands und/oder der Alterungsresistenz festgestellt werden, wenn die erste Kennzahl den ersten Referenzwert unterschreitet. Der Referenzwert kann einerseits aus theoretischen Überlegungen oder aus Erfahrungswerten von früheren Untersuchungen an Bitumenmaterialien mit unterschiedlichen Alterungsgraden gewonnen werden. Vorzugsweise wird der Referenzwert in einer Datenbank abgelegt. Bei der Durchführung des Verfahrens wird der Referenzwert aus der Datenbank abgerufen, um einen Vergleich mit der ersten Kennzahl zu ermöglichen, welcher aus den Messwerten der Fluoreszenzstrahlung bei unterschiedlichen Anregungswellenlängen ermittelt wurde.

Bei den Untersuchungen hat sich herausgestellt, dass die Alterung auf Basis der ersten Kennzahl allein nicht bei allen Bitumenproben zuverlässig festgestellt werden konnte. Es hat sich gezeigt, dass die erste Kennzahl bei manchen Bitumenproben unauffällig ist, obwohl die Materialeigenschaften durch komplexe Prozesse, wie beispielsweise Alterung bei Vorverwendung oder thermische Schädigung bei der Lagerung etc., bereits beeinträchtigt sind. Um diese Klasse von Bitumenmaterialien dem Verfahren zur Feststellung der Alterung zugänglich zu machen, werden bei einer bevorzugten Ausführung des Verfahrens die weiteren Schritte
- Beaufschlagen des Bitumenmaterials mit einer im Wesentlichen monochromatischen dritten Anregungsstrahlung einer dritten Wellenlänge;
- Messen der Intensität einer durch die dritte Anregungsstrahlung angeregten dritten Fluoreszenzstrahlung in dem Messwellenlängenbereich;
- Ermittlung einer zweiten Kennzahl für den Alterungszustand bzw. die Alterungsresistenz des Bitumens aus dem Verhältnis zwischen der Intensität der dritten Fluoreszenzstrahlung zu der Intensität der ersten Fluoreszenzstrahlung
durchgeführt.

Bei dieser Ausführung wird die Fluoreszenzemission des Bitumenmaterials aufgrund der Anregung mit der dritten Anregungsstrahlung der dritten Anregungswellenlänge erfasst, wobei die dritte Anregungswellenlänge sowohl von der ersten als auch von der zweiten Anregungswellenlänge verschieden ist. Die Intensität der dritten Fluoreszenzstrahlung, welche in einem zeitlichen Abstand zur ersten und zweiten Fluoreszenzstrahlung gemessen werden kann, wird anschließend ins Verhältnis zur Intensität der ersten Fluoreszenzstrahlung gesetzt, um eine zweite Kennzahl insbesondere für den Alterungszustand des Bitumenmaterials zu erhalten. Dadurch können alterungsbeständige Bitumenproben zuverlässig von solchen Bitumenproben unterschieden werden, deren erste Kennzahl über dem ersten Referenzwert liegt, aber dennoch tiefgreifende Alterungsprozesse durchlaufen haben. Dabei sind die Ursachen der Alterungsprozesse unwesentlich, welche in langfristigen Veränderungen während der Liegedauer eines Recyclingmaterials oder in einer Materialschädigung durch schlechte Lagerung oder Verarbeitung liegen können.

Um die Qualität des untersuchten Bitumenmaterials auf einfache Weise feststellen zu können, ist es von Vorteil, wenn die zweite Kennzahl mit einem zweiten Referenzwert verglichen wird. Bei dieser Ausführung kann daher die Erst- oder Wiederverwendung des Bitumenmaterials davon abhängig gemacht werden, dass die erste Kennzahl den ersten Referenzwert und die zweite Kennzahl den zweiten Referenzwert überschreitet.

Bei umfangreichen Versuchen zur Fluoreszenz von Bitumen wurde festgestellt, dass bestimmte Anregungswellenlängen besonders vorteilhaft sind, um die zu untersuchende Materialeigenschaft, insbesondere den Alterungszustand, des Bitumenmaterials zu ermitteln. Die erste Anregungswellenlänge der ersten Anregungsstrahlung ist vorzugsweise aus einem Wellenlängenbereich zwischen 260 und 280 Nanometer ausgewählt. Bei einer Anregung des Bitumens in dem genannten Wellenlängenbereich wird das globale Maximum der Fluoreszenz des Bitumens erhalten, dessen Intensität komplementäre Information über den Alterungszustand liefern kann. Allerdings kann die Intensität der beobachteten Emission stark vom Mess- bzw. Probenaufbau, insbesondere hinsichtlich Fokus und Abständen, beeinflusst sein. Diese Einflüsse können vor allem bei rauen Probenoberflächen problematisch sein. Das Verhältnis der Intensitäten ist hingegen im Wesentlichen unabhängig von der Mess- und Probengeometrie, sofern die Restintensität ausreichend ist. Insbesondere wurde in Untersuchungen gezeigt, dass das Verhältnis der Intensitäten der unterschiedlichen Fluoreszenzmaxima eine zuverlässige Aussage über die gesuchte Materialeigenschaft erlaubt. Demnach eignet sich die erste Anregungswellenlänge aus dem Bereich des globalen Maximums der Fluoreszenz besonders zur Verwendung als Bezugsgröße für den alterungsbedingten Intensitätsabfall der Fluoreszenz, welcher bei größeren Wellenlängen der Anregungsstrahlung feststellbar ist.

Gemäß einer besonders bevorzugten Ausführung ist vorgesehen, dass die zweite Anregungswellenlänge der zweiten Anregungsstrahlung aus einem Wellenlängenbereich zwischen 350 und 380 Nanometer ausgewählt ist. Bei Fluoreszenzanalysen konnte nachgewiesen werden, dass die Intensität der Fluoreszenzstrahlung zwischen 350 und 380 Nanometer ein lokales Maximum bzw. eine spektrale Schulter aufweist. Demnach wird die zweite Anregungswellenlänge aus einer lokalen Extremstelle der Fluoreszenz ausgewählt, bei welcher die Ableitungsfunktion des Fluoreszenzsignals im Wesentlichen den Wert Null einnimmt. Diese Ausführung hat einerseits den Vorteil, dass die alterungsbedingte Abweichung der Intensität der Fluoreszenzstrahlung im Bereich des lokalen Maximums besonders groß ist. Andererseits kann die Messgenauigkeit erhöht werden, da die Änderung des Fluoreszenzsignals im Bereich des lokalen Maximums gering ist.

Darüber hinaus ist es günstig, wenn die dritte Anregungswellenlänge der dritten Anregungsstrahlung aus einem Wellenlängenbereich zwischen 470 und 500 Nanometer ausgewählt ist. Das Fluoreszenzspektrum des Bitumenmaterials weist ein weiteres lokales Maximum in dem genannten Wellenlängenbereich auf, so dass es aus den zuvor genannten Gründen besonders vorteilhaft ist, die Fluoreszenz bei einer Anregung in diesem Wellenlängenbereich zu bestimmen.

In manchen Fällen kann es günstig sein, wenn die Intensität einer vierten Fluoreszenzstrahlung erfasst wird, welche mit einer im Wesentlichen monochromatischen vierten Anregungsstrahlung einer vierten Anregungswellenlänge zwischen 440 und 460 nm angeregt wird. In diesem Bereich liegt eine weitere spektrale Schulter des Fluoreszenzspektrums, welche für die Auswertung der Materialeigenschaft verwendet werden kann. Allerdings ist dieses lokale Maximum vergleichsweise schwach ausgeprägt und daher fehleranfälliger als die zuvor beschriebenen lokalen Maxima der Fluoreszenzstrahlung.

Bei umfangreichen Untersuchungen konnte nachgewiesen werden, dass die genannten Bereiche für die erste bzw. zweite Anregungswellenlänge charakteristisch sind für bestimmte Fraktionen des Bitumens, sogenannte Aromate und Harze, die einen wesentlichen Einfluss auf die strukturellen und mechanischen Eigenschaften von Bitumen nehmen. Durch die Untersuchung der Fluoreszenz bei Anregung des Bitumenmaterials mit den bevorzugten Werten der ersten bzw. zweiten Anregungswellenlänge können die Häufigkeit, Beständigkeit und chemische Resistenz gegen Oxidation der Aromate und Harze ermittelt werden. Zu diesem Zweck werden vorzugsweise die erste und die zweite Kennzahl berechnet, aus welchen die Alterungsbeständigkeit des untersuchten Bitumenmaterials abgeschätzt werden kann.

Gemäß einer bevorzugten Ausführungsform ist der Messwellenlängenbereich im Wesentlichen monochromatisch mit einer Messwellenlänge zwischen 390 und 650 Nanometer, insbesondere im Wesentlichen 525 Nanometer. Bei dieser Ausführung kann vorteilhafterweise ein maximales Signal erhalten werden.

Im Folgenden wird zum besseren Verständnis der Erfindung auch eine Vorrichtung beschrieben, die aber nicht vom Schutzumfang der Ansprüche umfasst ist. Die Vorrichtung zur Bestimmung des Alterungszustands und/oder der Alterungsresistenz eines Bitumenmaterials weist zumindest die folgenden Komponenten auf:
- eine erste Strahlungseinrichtung zum Beaufschlagen des Bitumenmaterials mit einer im Wesentlichen monochromatischen ersten Anregungsstrahlung einer ersten Anregungswellenlänge;
- eine zweite Strahlungseinrichtung zum Beaufschlagen des Bitumenmaterials mit einer im Wesentlichen monochromatischen zweiten Anregungsstrahlung einer zweiten Anregungswellenlänge;
- eine Messeinrichtung zum Messen der Intensität einer durch die erste Anregungsstrahlung angeregten ersten Fluoreszenzstrahlung in einem vorgegebenen Messwellenlängenbereich und zum Messen der Intensität einer durch die zweite Anregungsstrahlung angeregten zweiten Fluoreszenzstrahlung in dem vorgegebenen Messwellenlängenbereich;
- eine Recheneinrichtung mit einem ersten Verhältnisbilder zur Ermittlung einer ersten Kennzahl für den Alterungszustand bzw. die Alterungsresistenz des Bitumenmaterials aus dem Verhältnis zwischen der Intensität der zweiten Fluoreszenzstrahlung zu der Intensität der ersten Fluoreszenzstrahlung.

Die Vorrichtung bringt dieselben Vorteile mit sich wie das zuvor erläuterte Verfahren, so dass auf die vorstehenden Ausführungen verwiesen werden kann. Wesentlich ist, dass besonders einfache Strahlungseinrichtungen verwendet werden können, da die erste Kennzahl für den Alterungszustand bzw. die Alterungsresistenz, insbesondere die Alterung des Bitumenmaterials, aus lediglich zwei Fluoreszenzsignalen bestimmt wird, welche bei zwei diskreten Anregungswellenlängen aufgenommen werden. Demgegenüber ist es insbesondere nicht notwendig, die Strahlungs-, Mess- bzw. Recheneinrichtung dahingehend auszulegen, dass ein Anregungsspektrum über einen breiten Wellenlängenbereich der Anregungswellenlänge ausgewertet wird.

Um die für eine Erst- oder Wiederverwendung ungeeigneten Bitumenproben zu identifizieren, weist die Vorrichtung zur Bestimmung des Alterungszustands bzw. der Alterungsresistenz des Bitumenmaterials vorzugsweise zudem die folgenden Komponenten auf:
- eine dritte Strahlungseinrichtung zum Beaufschlagen des Bitumenmaterials mit einer im Wesentlichen monochromatischen dritten Anregungsstrahlung einer dritten Anregungswellenlänge, wobei die Messeinrichtung zum Messen der Intensität einer durch die dritte Anregungsstrahlung angeregten dritten Fluoreszenzstrahlung in dem vorgegebenen Messwellenlängenbereich eingerichtet ist;
- einen zweiten Verhältnisbilder zur Ermittlung einer zweiten Kennzahl für den Alterungszustand bzw. die Alterungsresistenz des Bitumenmaterials aus dem Verhältnis zwischen der Intensität der dritten Fluoreszenzstrahlung zu der Intensität der ersten Fluoreszenzstrahlung.

Um die Verwendbarkeit des Bitumenmaterials auf einfache Weise festzustellen, ist es vorteilhaft, wenn die Recheneinrichtung eine erste Datenbank mit einem ersten Referenzwert für den Alterungszustand bzw. die Alterungsresistenz des Bitumenmaterials und ein erstes Vergleichsmodul zum Vergleich der ersten Kennzahl mit dem ersten Referenzwert aufweist, wobei die Recheneinrichtung vorzugsweise eine zweite Datenbank mit einem zweiten Referenzwert für den Alterungszustand bzw. die Alterungsresistenz des Bitumenmaterials und ein zweites Vergleichsmodul zum Vergleich der zweiten Kennzahl mit dem zweiten Referenzwert aufweist. Wenn die erste Kennzahl den ersten Referenzwert unterschreitet, kann das Bitumenmaterial gemäß einer bevorzugten Anwendung des Verfahrens von einer Erst- oder Wiederverwendung bzw. Rezyklierung ausgeschlossen werden. Darüber hinaus wird eine Verwendung des Bitumenmaterials dann nicht ohne weitere Maßnahmen vorgenommen, wenn zwar die erste Kennzahl über dem ersten Referenzwert liegt, aber die zweite Kennzahl den zweiten Referenzwert unterschreitet. Nur wenn die erste Kennzahl den ersten Referenzwert und die zweite Kennzahl den zweiten Referenzwert überschreitet, weist das gebrauchte Bitumenmaterial die für die Verwendung gewünschten Materialeigenschaften auf, so dass der Auswahlprozess wesentlich verbessert werden kann.

Gemäß einer besonders bevorzugten Ausführung sind die erste und zweite Strahlungseinrichtung voneinander verschieden, wobei die erste Strahlungseinrichtung zum Aussenden der im Wesentlichen monochromatischen ersten Anregungsstrahlung der ersten Anregungswellenlänge und die zweite Strahlungseinrichtung zum Aussenden der im Wesentlichen monochromatischen Anregungsstrahlung der zweiten Anregungswellenlänge eingerichtet ist.

Zu diesem Zweck ist es günstig, wenn die erste Strahlungseinrichtung eine erste Leuchtdiode zum Aussenden der ersten Anregungsstrahlung mit der ersten Anregungswellenlänge und/oder die zweite Strahlungseinrichtung eine zweite Leuchtdiode zum Aussenden der zweiten Anregungsstrahlung mit der zweiten Anregungswellenlänge und/oder die dritte Strahlungseinrichtung eine dritte Leuchtdiode zum Aussenden der dritten Anregungsstrahlung mit der dritten Anregungswellenlänge aufweist. Alternativ zu den Leuchtdioden können Diodenlaser verwendet werden.

Gemäß einer weiteren bevorzugten Ausführung sind die erste und zweite Strahlungseinrichtung, gegebenenfalls auch die dritte Strahlungseinrichtung, durch eine gemeinsame Strahlungseinrichtung gebildet, welche eine Strahlungsquelle zum Aussenden einer breitbandigen Anregungsstrahlung aufweist. Bei dieser Ausführung ist zwischen der Strahlungsquelle für die breitbandige Anregungsstrahlung und dem Bitumenmaterial eine Einrichtung zur Auswahl der ersten, zweiten bzw. dritten Anregungswellenlänge vorgesehen, welche beispielsweise durch einen Filter oder einen Monochromator gebildet ist.

Um den Benutzer der Vorrichtung über den Alterungszustand des Bitumenmaterials zu informieren, ist es günstig, wenn die Vorrichtung zudem eine Anzeigeeinheit zur Anzeige der ersten und/oder der zweiten Kennzahl und/oder des ersten Referenzwertes und/oder des zweiten Referenzwertes für den Alterungszustand bzw. die Alterungsresistenz des Bitumenmaterials aufweist. Im Stand der Technik sind solche Anzeigeeinheiten, wie Displays, hinlänglich bekannt, so dass sich nähere Ausführungen hierzu erübrigen können.

Die der Erfindung zugrundeliegende Aufgabe wird andererseits durch ein Verfahren zur Bestimmung des Alterungszustands und/oder der Alterungsresistenz eines Bitumenmaterials, nämlich eines Straßenbaubitumens, mit den folgenden Schritten gelöst:
- Beaufschlagen des Bitumenmaterials mit einem im Wesentlichen monochromatischen Anregungslicht;
- Messen der Intensität eines durch das Anregungslicht angeregten ersten Fluoreszenzsignals bei einer ersten Emissionswellenlänge;
- Messen der Intensität eines durch das Anregungslicht angeregten zweiten Fluoreszenzsignals bei einer zweiten Emissionswellenlänge;
- Ermittlung einer ersten Kennzahl für den Alterungszustand bzw. die Alterungsresistenz des Bitumenmaterials aus dem Verhältnis zwischen der Intensität des zweiten Fluoreszenzsignals bei der zweiten Emissionswellenlänge zu der Intensität des ersten Fluoreszenzsignals bei der ersten Emissionswellenlänge.

Im Unterschied zur zuvor beschriebenen Ausführungsform der Erfindung werden bei dieser Ausführung einzelne bzw. diskrete Intensitätswerte des Emissionsspektrums (bei einer ersten und einer davon verschiedenen zweiten Emissionswellenlänge) aufgrund der Anregung mit einem im Wesentlichen monochromatischen Anregungslicht dazu verwendet, die erste Kennzahl, insbesondere für den Alterungszustand des Bitumenmaterials, zu bilden. Beide Ausführungen der Erfindung bringen den Vorteil mit sich, dass die Aufnahme eines vollständigen Spektrums, nämlich des Anregungsspektrums bei der ersten Ausführungsvariante bzw. des Emissionsspektrums bei der zweiten Ausführungsvariante der Erfindung, unterbleiben kann, da sich die Materialeigenschaft, insbesondere der Alterungszustand bzw. die Widerstandsfähigkeit gegenüber der Alterung, des Bitumenmaterials bereits aus den einzelnen Messwerten der Fluoreszenz zuverlässig bestimmen lässt.

Um die alterungsbedingte Verschlechterung der Materialeigenschaften des Bitumens zuverlässig festzustellen, kann das zuvor beschriebene Verfahren durch die weiteren Schritte
- Messen der Intensität eines durch das Anregungslicht angeregten dritten Fluoreszenzsignals bei einer dritten Emissionswellenlänge;
- Ermittlung einer zweiten Kennzahl für den Alterungszustand bzw. die Alterungsresistenz des Bitumens aus dem Verhältnis zwischen der Intensität des dritten Fluoreszenzsignals bei der dritten Emissionswellenlänge zu der Intensität des ersten Fluoreszenzsignals bei der ersten Emissionswellenlänge
auf vorteilhafte Weise erweitert werden.

Im Folgenden wird zum besseren Verständnis der Erfindung eine weitere Vorrichtung beschrieben, die aber nicht vom Schutzumfang der Ansprüche umfasst ist. Die Vorrichtung zur Bestimmung des Alterungszustands bzw. der Alterungsresistenz eines Bitumenmaterials weist gemäß der zuvor beschriebenen alternativen Lösung der an die Erfindung gestellten Aufgabe zumindest die folgenden Komponenten auf:
- eine Strahlungseinheit zum Beaufschlagen des Bitumenmaterials mit einem im Wesentlichen monochromatischen Anregungslicht;
- eine Messeinheit zum Messen der Intensität eines durch das Anregungslicht angeregten ersten Fluoreszenzsignals bei einer ersten Emissionswellenlänge und zum Messen der Intensität eines durch das Anregungslicht angeregten zweiten Fluoreszenzsignals bei einer zweiten Emissionswellenlänge;
- eine Recheneinheit zur Ermittlung einer ersten Kennzahl für den Alterungszustand bzw. die Alterungsresistenz des Bitumenmaterials aus dem Verhältnis zwischen der Intensität des zweiten Fluoreszenzsignals bei der zweiten Emissionswellenlänge zu der Intensität des ersten Fluoreszenzsignals bei der ersten Emissionswellenlänge.

Die Erfindung wird nachstehend anhand eines bevorzugten Ausführungsbeispiels, auf das sie jedoch nicht beschränkt sein soll, weiter erläutert. In der Zeichnung zeigen:
Fig. 1 schematisch eine Vorrichtung zur Bestimmung des Alterungszustands eines Bitumenmaterials;
Fig. 2 ein Ablaufdiagramm zur Veranschaulichung einer Ausführungsvariante des erfindungsgemäßen Verfahrens zur Bestimmung des Alterungszustands des Bitumenmaterials; und
Fig. 3 die Anregungsspektren je einer alterungsbeständigen und einer nicht-alterungsbeständigen Bitumenprobe.

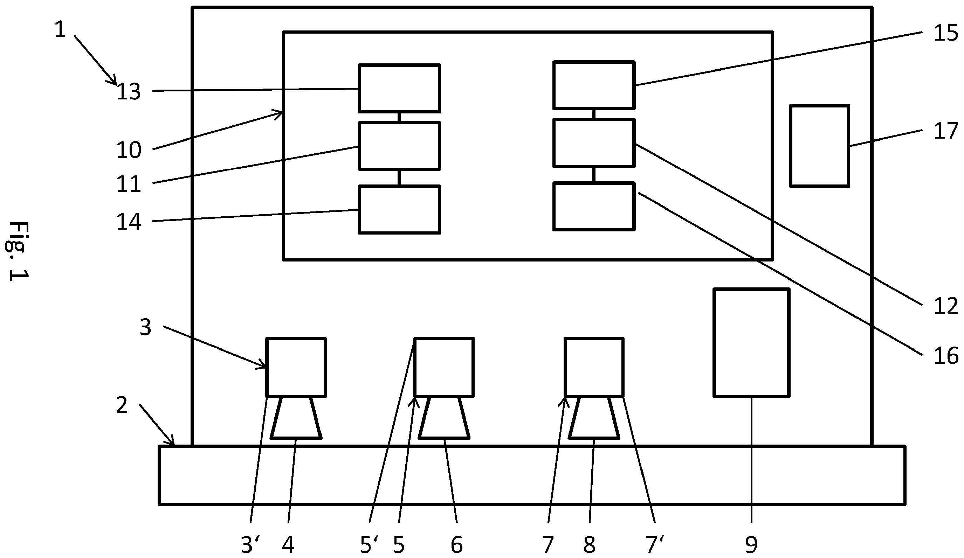

In Fig. 1 ist schematisch eine Vorrichtung 1 zur Bestimmung des Alterungszustands eines Bitumenmaterials 2, d.h. eines das Bindemittel Bitumen enthaltenden Materials, gezeigt. Das Bitumenmaterial 2 liegt insbesondere als Ausbauasphalt vor, dessen Alterungsbeständigkeit im Hinblick auf eine Rezyklierung untersucht werden soll.

Die Vorrichtung 1 weist eine erste Strahlungseinrichtung 3 zum Aussenden einer im Wesentlichen monochromatischen ersten Anregungsstrahlung 4 einer ersten Anregungswellenlänge λ1 auf. Darüber hinaus weist die Vorrichtung 1 eine zweite Strahlungseinrichtung 5 zum Aussenden einer im Wesentlichen monochromatischen zweiten Anregungsstrahlung 6 einer zweiten Anregungswellenlänge λ2 auf. Schließlich weist die Vorrichtung 1 eine dritte Strahlungseinrichtung 7 zum Aussenden einer im Wesentlichen monochromatischen dritten Anregungsstrahlung 8 einer dritten Anregungswellenlänge λ3 auf. Die Vorrichtung 1 weist ferner eine herkömmliche, im Stand der Technik an sich bekannte Messeinrichtung 9 auf, welche dazu eingerichtet ist, die Intensität I1 einer durch die erste Anregungsstrahlung 4 im Bitumenmaterial 2 angeregten ersten Fluoreszenzstrahlung, die Intensität I2 einer durch die zweite Anregungsstrahlung 6 angeregten zweiten Fluoreszenzstrahlung und die Intensität I3 einer durch die dritte Anregungsstrahlung 8 angeregten zweiten Fluoreszenzstrahlung zu messen. Die Fluoreszenz wird jeweils bei derselben, vorgegebenen Mess- bzw. Emissionswellenlänge von beispielsweise 515 Nanometer gemessen. Zu diesem Zweck werden die erste Strahlungseinrichtung 3, die zweite Strahlungseinrichtung 5 und die dritte Strahlungseinrichtung 7 in der gezeigten Ausführung zeitlich nacheinander aktiviert, wobei die Messeinrichtung 9 jeweils die vom Bitumenmaterial ausgehende erste Fluoreszenzstrahlung 4, zweite Fluoreszenzstrahlung 8 bzw. dritte Fluoreszenzstrahlung 8 erfasst. Alternativ zur Aktivierung der ersten 3, zweiten 5 und dritten Strahlungseinrichtung 7 in zeitlichem Abstand zueinander können wechselnde Filtersätze bzw. Blenden verwendet werden. Als erste Strahlungseinrichtung 3 ist in der gezeigten Ausführung eine erste Leuchtdiode 3', als zweite Strahlungseinrichtung 5 eine zweite Leuchtdiode 5' und als dritte Strahlungseinrichtung 7 eine dritte Leuchtdiode 7' vorgesehen.

Die einzelnen Komponenten der Vorrichtung 1 sind im Stand der Technik (vgl. zB CA 2 833 299, US 2005/0253088 A1, US 4,330,207, WO 2010/048584, US 7,633,071 B2) in verschiedenen Ausführungen bekannt, so dass sich nähere Ausführungen hierzu erübrigen können.

Wie aus Fig. 1 weiter ersichtlich, weist die Vorrichtung 1 zudem eine Recheneinrichtung 10 mit einem ersten Verhältnisbilder 11 zur Ermittlung einer ersten Kennzahl K1 für den Alterungszustand des Bitumenmaterials aus dem Verhältnis zwischen der Intensität I2 der zweiten Fluoreszenzstrahlung zu der Intensität I1 der ersten Fluoreszenzstrahlung auf. Darüber hinaus weist die Recheneinrichtung 10 einen zweiten Verhältnisbilder 12 zur Ermittlung einer zweiten Kennzahl K2 für den Alterungszustand des Bitumenmaterials 2 aus dem Verhältnis zwischen der Intensität I3 der dritten Fluoreszenzstrahlung zu der Intensität I1 der ersten Fluoreszenzstrahlung auf.

Um die Entscheidung über die Wiederverwendung des untersuchten Bitumenmaterial 2 vorzubereiten, weist die Recheneinrichtung 10 eine erste Datenbank 13 mit einem ersten Referenzwert R1 für den Alterungszustand des Bitumenmaterials 2 und ein erstes Vergleichsmodul 14 zum Vergleich der ersten Kennzahl K1 mit dem ersten Referenzwert R1 auf. Zudem weist die Recheneinrichtung 10 eine zweite Datenbank 15 mit einem zweiten Referenzwert R2 für den Alterungszustand des Bitumenmaterials und ein zweites Vergleichsmodul 16 zum Vergleich der zweiten Kennzahl mit dem zweiten Referenzwert auf. Der erste Referenzwert R1 und der zweite Referenzwert R2 sind charakteristisch für alterungsbeständige Bitumenmaterialien 2.

Wie aus Fig. 1 weiters ersichtlich, weist die Vorrichtung 1 zudem eine Anzeigeeinheit 17 zur Anzeige der ersten K1 und/oder der zweiten Kennzahl K2 und/oder des ersten Referenzwertes R1 und/oder des zweiten Referenzwertes R1 für den Alterungszustand des Bitumenmaterials 2 auf.

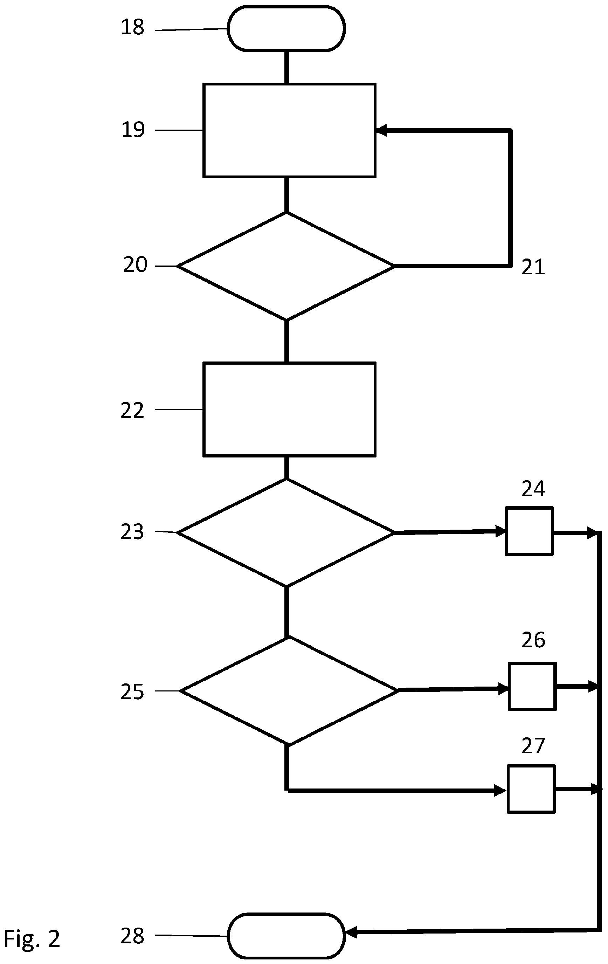

Das erfindungsgemäße Verfahren geht aus dem Ablaufdiagramm der Fig. 2 hervor. Nach dem Start 18 des Verfahrens wird nacheinander die Intensität I1 der ersten Fluoreszenzstrahlung, die Intensität I2 der zweiten Fluoreszenzstrahlung und die Intensität I3 der dritten Fluoreszenzstrahlung erfasst (Feld 19). Danach wird gemessen, ob die Intensität I1 der ersten Fluoreszenzstrahlung, die Intensität I2 der zweiten Fluoreszenzstrahlung und die Intensität I3 der dritten Fluoreszenzstrahlung einen Schwellwert Imin überschreitet (Feld 20). Sollte dies nicht der Fall sein, wird die Messung der Fluoreszenz wiederholt (Pfeil 21). Andernfalls wird die erste Kennzahl K1 als Verhältnis zwischen der Intensität I2 der zweiten Fluoreszenzstrahlung und der Intensität I1 der ersten Fluoreszenzstrahlung ermittelt (Feld 22). Danach wird die erste Kennzahl K1 mit dem ersten Referenzwert R1 verglichen (Feld 23). Ist die erste Kennzahl K1 kleiner als der erste Referenzwert R1, kann das Bitumenmaterial 2 als für die Rezyklierung ungeeignet erfasst werden (Feld 24). Wenn die erste Kennzahl K1 größer als der erste Referenzwert R1 ist, wird ein Vergleich zwischen der zweiten Kennzahl K2 und dem zweiten Referenzwert R2 vorgenommen (Feld 25). Unterschreitet die zweite Kennzahl K2 den zweiten Referenzwert R2, wird das Bitumenmaterial 2 als nicht alterungsbeständig betrachtet (Feld 26). Nur wenn auch die zweite Kennzahl K2 über dem zweiten Referenzwert R2 liegt, wird die Alterungsbeständigkeit des untersuchten Bitumenmaterials 2 angenommen (Feld 27). Solche Proben sind für eine Wiederverwendung geeignet. Feld 28 bezeichnet das Ende des Verfahrens.

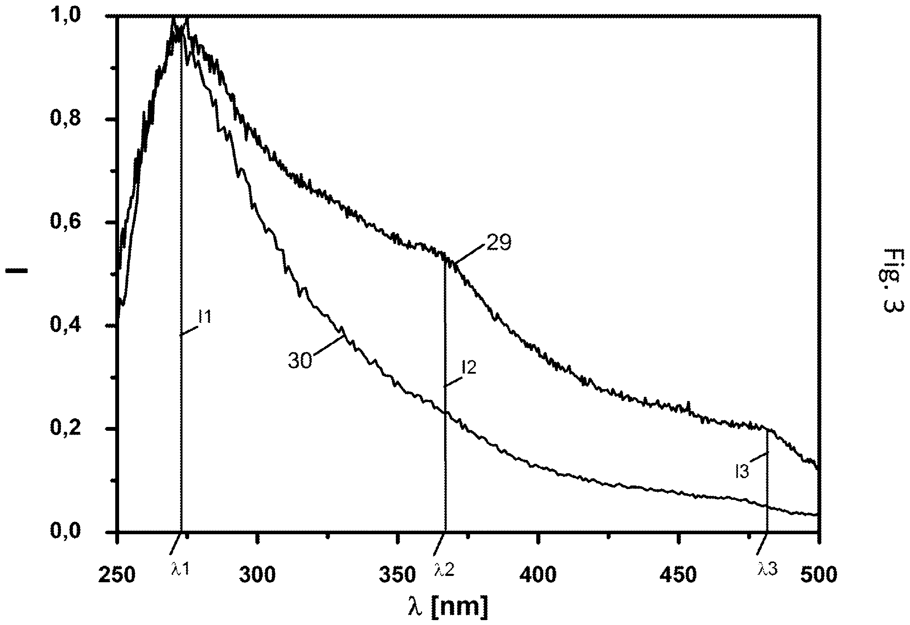

In Fig. 3 ist ein Diagramm mit den Anregungsspektren 29, 30 von zwei Bitumenproben 2 gezeigt, wobei sich das Anregungsspektrum 29 auf eine alterungsbeständige Bitumenprobe und das Anregungsspektrum 30 auf eine nicht alterungsbeständige Bitumenprobe bezieht. Auf der x-Achse ist die Wellenlänge λ der Anregungsstrahlung, auf der y-Achse die Intensität I der Fluoreszenz bei einer Messwellenlänge von 515 Nanometern (nm) aufgetragen. Daraus ist ersichtlich, dass die Fluoreszenz der gealterten Bitumenprobe bei Anregungswellenlängen λ oberhalb des Maximums bei ca. 270 nm gegenüber der alterungsbeständigen Bitumenprobe vermindert ist. Darüber hinaus weisen die Spektren charakteristische lokale Maxima bei ca. 370 nm und 480 nm auf.

Auf Basis dieser Erkenntnisse ist bevorzugt vorgesehen, dass die erste Anregungswellenlänge λ1 der ersten Anregungsstrahlung 4 aus einem Wellenlängenbereich zwischen 260 und 280 nm, insbesondere im Wesentlichen 270 nm, die zweite Anregungswellenlänge λ2 der zweiten Anregungsstrahlung 6 aus einem Wellenlängenbereich zwischen 350 und 380 nm, insbesondere im Wesentlichen 370 nm, und die dritte Anregungswellenlänge λ3 der dritten Anregungsstrahlung 8 aus einem Wellenlängenbereich zwischen 470 und 500 nm, insbesondere im Wesentlichen 480 nm, ausgewählt ist. Dadurch kann die erste Anregungswellenlänge λ1 als Referenz für den Intensitätsabfall bei größeren Anregungswellenlängen l herangezogen werden. Die Anregung im Bereich der lokalen Maxima des Anregungsspektrums ist aus messtechnischen Gründen besonders vorteilhaft.

Das erfindungsgemäße Prinzip kann zudem bei einer (nicht dargestellten) Vorrichtung verwirklicht sein, welche eine Strahlungseinheit zum Beaufschlagen des Bitumenmaterials mit einem im Wesentlichen monochromatischen Anregungslicht einer Anregungswellenlänge von beispielsweise 280 nm aufweist. Dadurch wird das Bitumenmaterial zu Fluoreszenz angeregt. Diese Ausführung der Vorrichtung 1 weist weiters eine Messeinheit auf, welche dazu eingerichtet ist, die Intensität eines durch das Anregungslicht angeregten ersten Fluoreszenzsignals bei einer ersten Emissionswellenlänge und die Intensität eines durch das Anregungslicht angeregten zweiten Fluoreszenzsignals bei einer zweiten Emissionswellenlänge zu messen. Die Vorrichtung weist zudem eine Recheneinheit zur Ermittlung einer ersten Kennzahl für den Alterungszustand des Bitumenmaterials aus dem Verhältnis zwischen der Intensität des zweiten Fluoreszenzsignals bei der zweiten Emissionswellenlänge zu der Intensität des ersten Fluoreszenzsignals bei der ersten Emissionswellenlänge auf. Entsprechend kann die zweite Kennzahl für den Alterungszustand des Bitumens aus dem Verhältnis zwischen der Intensität des dritten Fluoreszenzsignals bei der dritten Emissionswellenlänge zu der Intensität des ersten Fluoreszenzsignals bei der ersten Emissionswellenlänge gebildet werden.

## Patentansprüche

1. Verfahren zur Bestimmung des Alterungszustands bzw. der Alterungsresistenz eines Bitumenmaterials (2), mit den Schritten:
- Beaufschlagen des Bitumenmaterials (2) mit einer im Wesentlichen monochromatischen ersten Anregungsstrahlung (4) einer ersten Anregungswellenlänge (λ1);
- Messen der Intensität (I1) einer durch die erste Anregungsstrahlung (4) angeregten ersten Fluoreszenzstrahlung in einem Messwellenlängenbereich;
- Beaufschlagen des Bitumenmaterials (2) mit einer im Wesentlichen monochromatischen zweiten Anregungsstrahlung (6) einer zweiten Anregungswellenlänge (λ2), wobei die zweite Anregungswellenlänge (λ2) von der ersten Anregungswellenlänge (λ1) verschieden ist;
- Messen der Intensität (I2) einer durch die zweite Anregungsstrahlung (6) angeregten zweiten Fluoreszenzstrahlung in dem Messwellenlängenbereich;
- Ermittlung einer ersten Kennzahl (K1) für den Alterungszustand bzw. die Alterungsresistenz des Bitumenmaterials (2) aus dem Verhältnis zwischen der Intensität (I2) der zweiten Fluoreszenzstrahlung zu der Intensität (I1) der ersten Fluoreszenzstrahlung.

2. Verfahren nach Anspruch 1, **dadurch gekennzeichnet, dass** die erste Kennzahl (K1) mit einem ersten Referenzwert (R1) für den Alterungszustand bzw. die Alterungsresistenz des Bitumenmaterials (2) verglichen wird.

3. Verfahren nach Anspruch 1 oder 2, **gekennzeichnet durch** die weiteren Schritte
- Beaufschlagen des Bitumenmaterials (2) mit einer im Wesentlichen monochromatischen dritten Anregungsstrahlung (8) einer dritten Anregungswellenlänge (λ3), wobei die dritte Anregungswellenlänge (λ3) sowohl von der ersten (λ1) als auch von der zweiten Anregungswellenlänge (λ2) verschieden ist;
- Messen der Intensität (I3) einer durch die dritte Anregungsstrahlung (8) angeregten dritten Fluoreszenzstrahlung in dem Messwellenlängenbereich;
- Ermittlung einer zweiten Kennzahl (K2) für den Alterungszustand bzw. die Alterungsresistenz des Bitumenmaterials (2) aus dem Verhältnis zwischen der Intensität (I3) der dritten Fluoreszenzstrahlung zu der Intensität (I1) der ersten Fluoreszenzstrahlung.

4. Verfahren nach Anspruch 3, **dadurch gekennzeichnet, dass** die zweite Kennzahl (K2) mit einem zweiten Referenzwert (R2) verglichen wird.

5. Verfahren nach Anspruch 3 oder 4, **dadurch gekennzeichnet, dass** die dritte Anregungswellenlänge (λ3) der dritten Anregungsstrahlung (8) aus einem Wellenlängenbereich zwischen 470 und 500 Nanometer ausgewählt ist.

6. Verfahren nach einem der Ansprüche 1 bis 5, **dadurch gekennzeichnet, dass** die erste Anregungswellenlänge (λ1) der ersten Anregungsstrahlung (4) aus einem Wellenlängenbereich zwischen 260 und 280 Nanometer ausgewählt ist.

7. Verfahren nach einem der Ansprüche 1 bis 6, **dadurch gekennzeichnet, dass** die zweite Anregungswellenlänge (λ2) der zweiten Anregungsstrahlung (6) aus einem Wellenlängenbereich zwischen 350 und 380 Nanometer ausgewählt ist.

8. Verfahren nach einem der Ansprüche 1 bis 6, **dadurch gekennzeichnet, dass** der Messwellenlängenbereich im Wesentlichen monochromatisch mit einer Messwellenlänge zwischen 390 und 650 Nanometer, insbesondere im Wesentlichen 525 Nanometer, ist.

9. Verfahren zur Bestimmung des Alterungszustands bzw. der Alterungsresistenz eines Bitumenmaterials (2), wobei das Bitumenmaterial (2) ein Straßenbaubitumen ist, mit den Schritten:
- Beaufschlagen des Bitumenmaterials (2) mit einem im Wesentlichen monochromatischen Anregungslicht;
- Messen der Intensität eines durch das Anregungslicht angeregten ersten Fluoreszenzsignals bei einer ersten Emissionswellenlänge;
- Messen der Intensität eines durch das Anregungslicht angeregten zweiten Fluoreszenzsignals bei einer zweiten Emissionswellenlänge;
- Ermittlung einer ersten Kennzahl (K1) für den Alterungszustands bzw. die Alterungsresistenz des Bitumenmaterials (2) aus dem Verhältnis zwischen der Intensität des zweiten Fluoreszenzsignals bei der zweiten Emissionswellenlänge zu der Intensität des ersten Fluoreszenzsignals bei der ersten Emissionswellenlänge.

## Claims

1. Method for determining the ageing state or the ageing resistance of a bitumen material (2), comprising the following steps:
- exposing the bitumen material (2) to a substantially monochromatic first excitation radiation (4) of a first excitation wavelength (λ1);
- measuring the intensity (I1) of a first fluorescence radiation excited by the first excitation radiation (4) in a measurement wavelength range;
- exposing the bitumen material (2) to a substantially monochromatic second excitation radiation (6) of a second excitation wavelength (λ2), wherein the second excitation wavelength (λ2) is different from the first excitation wavelength (λ1);
- measuring the intensity (I2) of a second fluorescence radiation excited by the second excitation radiation (6) in the measurement wavelength range;
- determining a first characteristic number (K1) for the ageing state or the ageing resistance of the bitumen material (2) from the ratio of the intensity (I2) of the second fluorescent radiation to the intensity (I1) of the first fluorescent radiation.

2. Method according to claim 1, **characterised in that** the first characteristic number (K1) is compared with a first reference value (R1) for the ageing state or the ageing resistance of the bitumen material (2).

3. Method according to claim 1 or 2, **characterised by** the following additional steps:
- exposing the bitumen material (2) to a substantially monochromatic third excitation radiation (8) of a third excitation wavelength (λ3), the third excitation wavelength (λ3) being different from both the first (λ1) and the second excitation wavelength (λ2);
- measuring the intensity (I3) of a third fluorescence radiation excited by the third excitation radiation (8) in the measurement wavelength range;
- determining a second characteristic number (K2) for the ageing state or the ageing resistance of the bitumen material (2) from the ratio of the intensity (I3) of the third fluorescent radiation to the intensity (I1) of the first fluorescent radiation.

4. Method according to claim 3, **characterised in that** the second characteristic number (K2) is compared with a second reference value (R2).

5. Method according to claim 3 or 4, **characterised in that** the third excitation wavelength (λ3) of the third excitation radiation (8) is selected from a wavelength range of between 470 and 500 nanometers.

6. Method according to any of claims 1 to 5, **characterised in that** the first excitation wavelength (λ1) of the first excitation radiation (4) is selected from a wavelength range of between 260 and 280 nanometers.

7. Method according to any of claims 1 to 6, **characterised in that** the second excitation wavelength (λ2) of the second excitation radiation (6) is selected from a wavelength range of between 350 and 380 nanometers.

8. Method according to any of claims 1 to 6, **characterised in that** the measurement wavelength range is substantially monochromatic and has a measurement wavelength of between 390 and 650 nanometers, in particular substantially 525 nanometers.

9. Method for determining the ageing state or the ageing resistance of a bitumen material (2), wherein the bitumen material (2) is a road building bitumen, comprising the following steps:
- exposing the bitumen material (2) to a substantially monochromatic excitation light;
- measuring the intensity of a first fluorescence signal excited by the excitation light at a first emission wavelength;
- measuring the intensity of a second fluorescence signal excited by the excitation light at a second emission wavelength;
- determining a first characteristic number (K1) for the ageing state or the ageing resistance of the bitumen material (2) from the ratio of the intensity of the second fluorescence signal at the second emission wavelength to the intensity of the first fluorescence signal at the first emission wavelength.

## Revendications

1. Procédé de détermination de l'état de vieillissement et/ou de la résistance au vieillissement d'un matériau bitumineux (2), comprenant les étapes de :
- soumission du matériau bitumineux (2) à un premier rayonnement d'excitation (4) sensiblement monochromatique d'une première longueur d'onde d'excitation (λ1) ;
- mesure de l'intensité (I1) d'un premier rayonnement de fluorescence excité par le premier rayonnement d'excitation (4) dans une plage de longueurs d'ondes de mesure ;
- soumission du matériau bitumineux (2) à un deuxième rayonnement d'excitation (6) sensiblement monochromatique d'une deuxième longueur d'onde d'excitation (λ2), la deuxième longueur d'onde d'excitation (λ2) étant différente de la première longueur d'onde d'excitation (λ1) ;
- mesure de l'intensité (12) d'un deuxième rayonnement de fluorescence excité par le deuxième rayonnement d'excitation (6) dans la plage de longueurs d'ondes de mesure ;
- détermination d'un premier nombre caractéristique (K1) pour l'état de vieillissement et/ou la résistance au vieillissement du matériau bitumineux (2) à partir du rapport entre l'intensité (12) du deuxième rayonnement de fluorescence et l'intensité (I1) du premier rayonnement de fluorescence.

2. Procédé selon la revendication 1, **caractérisé en ce que** le premier nombre caractéristique (K1) est comparé à une première valeur de référence (R1) pour l'état de vieillissement et/ou la résistance au vieillissement du matériau bitumineux (2).

3. Procédé selon la revendication 1 ou 2, **caractérisé par** les autres étapes de
- soumission du matériau bitumineux (2) à un troisième rayonnement d'excitation (8) sensiblement monochromatique d'une troisième longueur d'onde d'excitation (λ3), la troisième longueur d'onde d'excitation (λ3) étant différente aussi bien de la première (λ1) que de la deuxième longueur d'onde d'excitation (λ2) ;
- mesure de l'intensité (13) d'un troisième rayonnement de fluorescence excité par le troisième rayonnement d'excitation (8) dans la plage de longueurs d'ondes de mesure ;
- détermination d'un deuxième nombre caractéristique (K2) pour l'état de vieillissement et/ou la résistance au vieillissement du matériau bitumineux (2) à partir du rapport entre l'intensité (13) du troisième rayonnement de fluorescence et l'intensité (I1) du premier rayonnement de fluorescence.

4. Procédé selon la revendication 3, **caractérisé en ce que** le deuxième nombre caractéristique (K2) est comparé à une deuxième valeur de référence (R2).

5. Procédé selon la revendication 3 ou 4, **caractérisé en ce que** la troisième longueur d'onde d'excitation (λ3) du troisième rayonnement d'excitation (8) est choisie parmi une plage de longueurs d'ondes entre 470 et 500 nanomètres.

6. Procédé selon l'une des revendications 1 à 5, **caractérisé en ce que** la première longueur d'onde d'excitation (λ1) du premier rayonnement d'excitation (4) est choisie parmi une plage de longueurs d'ondes entre 260 et 280 nanomètres.

7. Procédé selon l'une des revendications 1 à 6, **caractérisé en ce que** la deuxième longueur d'onde d'excitation (λ2) du deuxième rayonnement d'excitation (6) est choisie parmi une plage de longueurs d'ondes entre 350 et 380 nanomètres.

8. Procédé selon l'une des revendications 1 à 6, **caractérisé en ce que** la plage de longueur d'ondes de mesure est sensiblement monochromatique avec une longueur d'ondes de mesure entre 390 et 650 nanomètres, en particulier sensiblement de 525 nanomètres.

9. Procédé de détermination de l'état de vieillissement et/ou de la résistance au vieillissement d'un matériau bitumineux (2), le matériau bitumineux (2) étant un bitume routier, comprenant les étapes de :
- soumission du matériau bitumineux (2) à une lumière d'excitation sensiblement monochromatique ;
- mesure de l'intensité d'un premier signal de fluorescence excité par la lumière d'excitation pour une première longueur d'onde d'émission ;
- mesure de l'intensité d'un deuxième signal de fluorescence excité par la lumière d'excitation pour une deuxième longueur d'onde d'émission ;
- détermination d'un premier nombre caractéristique (K1) pour l'état de vieillissement et/ou la résistance au vieillissement du matériau bitumineux (2) à partir du rapport entre l'intensité du deuxième signal de fluorescence pour la deuxième longueur d'onde d'émission et l'intensité du premier signal de fluorescence pour la première longueur d'onde d'émission.
